# EUROPÄISCHE PATENTSCHRIFT

(11) **EP 2 197 836 B1**
(45) Veröffentlichungstag und Bekanntmachung des Hinweises auf die Patenterteilung: **24.04.2013**
(21) Anmeldenummer: 08803255.2
(22) Anmeldetag: 27.08.2008
(51) Int. Cl.: C07C 263/10, C07C 263/20, C07C 265/14

(54) **VERFAHREN ZUR HERSTELLUNG VON ISOCYANATEN**
METHOD FOR THE PRODUCTION OF ISOCYANATES
PROCÉDÉ DE PRODUCTION D'ISOCYANATES

(30) Priorität: 31.08.2007 EP 07115380
(43) Veröffentlichungstag der Anmeldung: 23.06.2010
(73) Patentinhaber: BASF SE, 67056 Ludwigshafen (DE)
(72) Erfinder: RUMPF, Bernd, 68766 Hockenheim (DE); BOCK, Michael, 67152 Ruppertsberg (DE); FIENE, Martin, 67150 Niederkirchen (DE); STROEFER, Eckhard, 68163 Mannheim (DE)
(86) Internationale Anmeldenummer: PCT/EP2008/061188
(87) Internationale Veröffentlichungsnummer: WO 2009/027418

(56) Entgegenhaltungen:
- EP-A- 1 717 223
- WO-A-2004/056757
- DE-A1- 19 827 852

## Beschreibung

Di- und polyfunktionelle Isocyanate, im folgenden auch als Polyisocyanate bezeichnet, wie Toluylendiisocyanat (TDI), Diphenylmethandiisocyanat (MDI), Isophorondiisocyanat (IPDI)oder Hexamethylendiisocyanat (HDI) sind wertvolle Ausgangsverbindungen für die Herstellung von Polyurethanen.

Di- und Polyisocyanate sowie deren Herstellung sind seit langem bekannt und vielfach beschrieben. Bekannte Verfahren zur Herstellung von Isocyanaten wie TDI, MDI, IPDI oder HDI beruhen zumeist auf einer Phosgenierung des entsprechenden Amins mit einer nachfolgenden Abtrennung des Chlorwasserstoffs und des überschüssigen Phosgens. Das Roh-Isocyanat/Lösungsmittelgemisch wird dann einer mehrstufigen Aufarbeitung unterzogen, um das Lösungsmittel sowie störende Leicht- und Schwersieder abzutrennen. Als Lösungsmittel für die Herstellung der Isocyanate werden vorzugsweise chlorierte aromatische Kohlenwasserstoffe wie Chlorbenzol, Dichlorbenzol, Trichlorbenzol oder aromatische Kohlenwasserstoffe wie Toluol, Xylol oder Benzol eingesetzt. Verschiedene, großtechnisch ausgeübte Verfahren zur Herstellung von Isocyanaten sind beispielsweise in Ullmanns Enzyklopädie der Technischen Chemie beschrieben.

Nachteilig an der zuvor beschriebenen Vorgehensweise ist aber, dass im Verlaufe der Destillation des Reinproduktes vergleichsweise große Verweilzeiten vorliegen, wodurch eine Schwersiederbildung aus dem Wertprodukt gefördert wird. Weiterhin gelangen im Rahmen der zuvor beschriebenen Vorgehensweise bereits in der Reaktion entstandene, hochsiedende Nebenprodukte in die Aufarbeitung, wodurch eine weitere Schwersiederbildung aus dem Wertprodukt Isocyanat gefördert wird.

Dementsprechend beträgt die Ausbeute beispielsweise im Verfahren zur Herstellung von TDI aus Toluylendiamin (TDA) zumeist nur etwa 95%.

Diese Nebenkomponenten haben die Eigenschaft, mit den Isocyanaten zu reagieren und somit den Anteil von Isocyanaten in einem Isocyanat / Nebenproduktgemisch zu reduzieren. Die schwere Handhabbarkeit und die typische Zusammensetzung eines solchen Rückstands wird beispielsweise in DE 102 60 093 A1 benannt.

In WO 2004/04516759 A1 wird beispielsweise die zweistufige Abtrennung von Isocyanaten aus einem Isocyanat/Schwersiedergemisch (Strom 1) beschrieben. Die Ströme 2 (Sumpf) und 3 (Destillat) werden im Gewichtsverhältnis 20:1 bis 1:1 aufgeteilt. D.h. es werden höchstens 50% des Stromes 1 über Sumpf gezogen. Die Lösung wird aufkonzentriert , in einen Kneter gepumpt, und dort weiter eingedampft.

Aufgabe der vorliegenden Erfindung war es, ein Verfahren zur Herstellung von Polyisocyanaten zu entwickeln, das mit einer hohen Ausbeute betrieben werden kann und das insbesondere die bei der Aufarbeitung des rohen Isocyanatgemisches resultierenden Ausbeuteverluste vermeidet.

Es wurde nun erstaunlicherweise gefunden, dass die genannten Ausbeuteverluste wirksam vermindert werden können, wenn man die bereits im Roh-Isocyanatgemisch vorliegenden, schwersiedenden Verbindungen wie z. B. Harnstoffe und seine durch Phosgenierung entstandenen Folgeprodukte, d.h. Carbodiimide, Isocyanurate, Uretdione vor oder während der eigentlichen Destillationssequenz zur Abtrennung des Lösungsmittels und der Leichtsieder mit einem geeigneten Apparatekonzept abtrennt. Des weiteren wurde gefunden, dass durch eine geeignete Voreindampfung Teile der schwersiedenden, das Isocyanat enthaltenden Komponenten in das Isocyanat zurückgespalten werden können, wodurch einerseits Ausbeuteverluste über Sumpf wirksam vermindert werden können, andererseits aber, entgegen der bisherigen Lehre ,das Sumpfprodukt selbst bei hohen Abdampfraten fließfähig und damit gut förderbar bleibt. Weiterhin wird dadurch vermieden, dass sich monomeres Isocyanat bei der Aufarbeitung an die schwersiedenden Verbindungen anlagert und dadurch die Ausbeute reduziert wird.

Gegenstand der Erfindung ist demzufolge ein Verfahren zur Herstellung von Polyisocyanaten, umfassend die Schritte
a) Umsetzung von Aminen mit Phosgen,
b) Abtrennung von Chlorwasserstoff, überschüssigem Phosgen und gegebenenfalls des Lösungsmittels aus dem Reaktionsgemisch,
c) Trennung des flüssigen Gemisches aus Schritt b) in eine flüssige und eine gasförmige Phase in einem Wendelrohrverdampfer.
d) Aufarbeitung der gasförmigen Phase aus Schritt c) zum Polyisocyanat.

In US 3140305 ist beispielsweise die Verwendung eines Dünnschichtverdampfers zur Abtrennung der in der Reaktion entstandenen Schwersieder beschrieben. Nachteilig am beschriebenen Verfahren ist jedoch das vergleichsweise aufwendige und in der Anschaffung sowie im Betrieb, beispielsweise aufgrund von erhöhtem Instandhaltungsbedarf, kostenintensive Apparatekonzept. Denkbar ist beispielsweise auch der Einsatz von Fallfilmverdampfern. Allerdings ist bei Einsatz eines Fallfilmverdampfers zu erwarten, dass der Anteil an Nebenprodukten aufgrund des erforderlichen Betriebes des Apparates mit einem Umpumpkreis und der daraus resultierenden Verweilzeiten deutlich größer als in einem Dünnschichtverdampfer ist. Weiterhin können Fallfilmverdampfer nur bis zu gewissen Grenzviskositäten betrieben werden, sodass die Menge an Sumpfprodukt und damit der Verlust an Isocyanaten deutlich größer sein wird.

Die genannten Nachteile können durch den Einsatz eines Wendelrohrverdampfers vermieden werden.

Schritt c) wird daher in einem Wendelrohrverdampfer durchgeführt. Der Wendelrohrverdampfer wird beispielsweise in DE 198 27 852 A1, sowie in Chem. Ing. Tech (68), 1996, S. 706-710, beschrieben. Der Wendelrohrverdampfer ist ein gewendeltes Rohr mit mehreren Windungen, in dem die Aufkonzentrierung einer Lösung durch Beheizung des Rohres von außen durchgeführt wird. Am Eintritt wird durch Überhitzung des Zulaufs und Entspannung ein zweiphasiges Gemisch in den Wendelrohrverdampfer gegeben. Durch die Verdampfung im gewendelten Rohr wird schnell eine zweiphasige Strömung hoher Strömungsgeschwindigkeit erzeugt, wodurch sehr hohe Wärmeübergangskoeffizienten erzielt werden können. Weiterhin wird, bedingt durch die hohen Scherkräfte an der Wand, eine weitgehende Selbstreinigung des Rohres erzielt. Die Verweilzeit im Apparat ist sehr gering. Am Austritt wird die Zweiphasenströmung in einem Abscheider, beispielsweise Schwerkraftabscheider oder Zentrifugalabscheider, getrennt.

Der Druck nach dem Apparat für Schritt c), das heißt im Abscheider, beträgt vorzugsweise 5 bis 200 mbar, insbesondere 5 bis 30 mbar.

Die Temperatur am Austritt des Apparats für Schritt c), das heißt im Abscheider, beträgt vorzugsweise 100 bis 300 °C, insbesondere 130 bis 250 °C.

In einer vorteilhaften Ausführungsform des erfindungsgemäßen Verfahrens wird das Gemisch aus Schritt b) vor dem Schritt c) unter Druck vorgewärmt und bei Eintritt in den Apparat für Schritt c) entspannt. Dies erfolgt in einem Apparat, der im folgenden als Vorwärmer bezeichnet wird. Der Vorwärmer ist vorzugsweise ein Wärmeaustauscher. Als Vorwärmer eignen sich beispielsweise Rohrbündelapparate, Plattenapparate, Spiralwärmeübertrager oder Doppelrohrapparate. Die Auswahl, Auslegung sowie die Bauweise derartiger Apparate ist dem Fachmann bekannt.

Die Beheizung des Vorwärmers und des Wendelrohrverdampfers kann mit Dampf erfolgen, ebenso ist die Beheizung mit Wärmeträgem wie z. B. Thermalölen denkbar. Weiterhin ist auch eine im Gesamtprozess wärmeintegrierte Fahrweise denkbar.

Die Bedingungen im Vorwärmer sollten dabei so gewählt werden, dass an keiner Stelle im Vorwärmer Verdampfung eintritt. Beim Eintritt des überhitzten Gemisches aus dem Vorwärmer in den Apparat von Schritt c) wird dieses entspannt, wodurch ein zweiphasiges Gemisch entsteht. Dieses wird dem Apparat aus Schritt c) zugeleitet und dort durch weitere Wärmezufuhr eingedampft. Insbesondere bei Verwendung eines Wendelrohrverdampfers kann durch Wahl der Geometrie, des Gesamtmengenstromes und des Gasanteiles nach der Entspannung im Rohr eine wellige Filmströmung eingestellt werden. Dadurch kommt es zu einer intensiven Durchmischung des Flüssigkeitsfilmes, sodass Temperatur- und Konzentrationsgradienten im Film wirksam abgebaut werden. Weiterhin liegen hohe Schubspannungen im Bereich der Wand vor, sodass der Aufbau von Belägen an den beheizten Wänden wirkungsvoll vermieden wird.

Die zu erreichende Abdampfrate und damit die Konzentration des Wertproduktes im Sumpfprodukt wird üblicherweise durch die Wahl der Beheizungstemperatur und des Druckes am Austritt von Schritt c), vorzugsweise im Abscheider, festgelegt und kann beispielsweise durch Versuche ermittelt werden.

Nach dem Schritt c) werden Dampf und Flüssigkeit voneinander getrennt. Dies kann beispielsweise in einem nachgeschalteten Abscheider geschehen. Die Brüden können beispielsweise in einem dem Abscheider nachgeschalteten Kondensator kondensiert und dem Schritt d), insbesondere der Reindestillation, zugeleitet werden. Dort wird die Mischung zum reinen Isocyanat aufgearbeitet.

Prinzipiell ist es ebenso möglich, die Brüden ohne weiteren Kondensationsschritt direkt in die Reindestillation von Schritt d) zu überführen.

Der Austrag des je nach Abdampfrate viskosen Sumpfproduktes von Verfahrensschritt c) kann beispielsweise mit Hilfe von großmauligen Zahnradpumpen erfolgen

Zumeist kann, je nach erzielter Abdampfrate, das Sumpfprodukt, das heißt die flüssige Phase, noch Isocyanat und rückspaltbare Anteile des Isocyanats, das sind insbesondere an den hochmolekularen Rückstand kovalent gebundene Uretdione und Uretonimine, enthalten. Die dadurch entstehenden Ausbeuteverluste können dadurch reduziert werden, dass man das Sumpfprodukt thermisch behandelt, insbesondere in einem weiteren Eindampfschritt behandelt. Dies kann beispielsweise durch eine erneute Eindampfung in einem Wendelrohrverdampfer erfolgen. Denkbar ist auch der Einsatz von mechanisch gewischten Apparaten wie Dünnschichtverdampfem oder Kurzwegverdampfem. Dabei kann es erforderlich sein, dem erhaltenen Rückstand bzw. dem Sumpfprodukt aus der ersten Eindampfstufe Fließhilfsmittel zuzusetzen. So beschreibt DE 41 42 769 A1 den Einsatz von Bitumen und DE 41 27 514 A1 den Einsatz von MDI oder PMDI als Fließhilfsmittel. Ebenso sind hier die sogenannten List-Kneter bzw. List-Entgaser grundsätzlich denkbar. Gegebenfalls kann zwischen oder nach einem oder beiden Eindampfschritten ein Verweilzeitraum, beispielsweise ein Behälter, Schaufeltrockner, Rührkessel, Rohrreaktor, Wendelrohrverdampfer, Wärmetauscher, Kolonnensumpf, Extruder oder Kneter, insbesondere ein Wendelrohrverdampfer, zur Förderung der Rückspaltung vorgesehen werden. Die Verweilzeit beträgt bei den vorbenannten Beispielen vorzugsweise 0,5-5 h bei Temperaturen von 120-250°C, insbesondere 159-240 °C. und Drücken je nach Fließhilfsmittel von 1 mbar - 10 bar.

Das Sumpfprodukt kann aus dem Verfahren ausgeschleust und einer Verwertung zugeführt werden. Dies ist jedoch weniger bevorzugt, da bei dieser Ausführungsform auch im Sumpfprodukt enthaltendes reines beziehungsweise aus diesem leicht rückgewinnbares Isocyanat mit ausgeschleust wird.

Die verbleibenden Sumpfprodukte, zumeist in polymer Form vorliegenden Carbodiimide und mehrkernige chlorierte Nebenprodukte, können beispielsweise deponiert oder verbrannt werden. Bevorzugt ist es möglich, sie in einem nächsten Schritt zum entsprechenden Amin zu hydrolysieren. Das zurückgewonnene Amin kann vorzugsweise in die Phosgenierung zurückgeführt oder mit Alkylenoxiden zu Polyetheralkoholen umgesetzt werden.

Wie beschrieben, kann das erfindungsgemäße Verfahren bei allen durch Phosgenierung hergestellten lasocyanaten angewandt werden. Bevorzugt wird es bei der Herstellung von Toluylendiisocyanat (TDI), Diphenylmethandiisocyanat (MDI), Isophorondiisocyanat (IPDI)oder Hexamethylendiisocyanat (HDI), insbesondere bei der Herstellung von TDI eingesetzt.

Das Grundprinzip des erfindungsgemäßen Verfahrens unter Verwendung eines Wendelrohrverdampfers kann in allgemeiner Form wie folgt dargestellt werden. Die Roh-Isocyanatlösung wird mittels einer Pumpe in einen Vorwärmer gefördert und dort unter Druck vorgewärmt. Dazu wird der Druck an einem Druckhalteventil so eingestellt, das an keiner Stelle im Vorwärmer Verdampfung eintritt. Das zum Druck vor dem Wendelrohrverdampfer überhitzte Gemisch wird am Druckhalteventil entspannt, wodurch ein zweiphasiges Gemisch entsteht. Das Gemisch wird dem Wendelrohrverdampfer zugeleitet und dort durch weitere Wärmezufuhr eingedampft. Dadurch kommt es zu einer intensiven Durchmischung des Flüssigkeitsfilmes, sodass Temperatur- und Konzentrationsgradienten im Film wirksam abgebaut werden. Weiterhin liegen hohe Schubspannungen im Bereich der Wand vor, sodass der Aufbau von Belägen an den beheizten Wänden wirkungsvoll vermieden wird. Die zu erreichende Abdampfrate und damit die Konzentration des Wertproduktes im Sumpfprodukt wird durch die Wahl der Beheizungstemperatur festgelegt und kann beispielsweise durch Versuche ermittelt werden. Im nachgeschalteten Abscheider (5) werden Dampf und Flüssigkeit voneinander getrennt, die Brüden werden im Kondensator kondensiert und nach dem Sammler der Reindestillation zugeleitet.

Die Eindampfung erfolgt bei Drücken im Abscheider von 2 bis 100 mbar, bevorzugt bei Drücken von 5 bis 30 mbar. Die Austrittstemperaturen aus dem Vorwärmer und dem Wendelrohrverdampfer betragen 100 bis 300 Grad, bevorzugt 130 bis 250 Grad.

Die Abdampfrate (=Mengenstrom der Brüden bezogen auf den Mengenstrom des zulaufenden Stromes) beträgt bevorzugt 85 bis 99 Gew.-%, besonders bevorzugt 90 bis 98 Gew.-%.

Die Erfindung soll an den nachstehenden Beispielen näher erläutert werden.

### Beispiel 1 (nicht erfindungsgemäß):

TDI wurde aus TDA und Phosgen synthetisiert. Der Reaktionsaustrag enthielt 77% TDI, 20% Chlorbenzol und 3% nicht verdampfbaren Rückstand. Das Lösungsmittel Toluol wurde in den Kolonnen K1 (Sumpftemperatur 160°C) und K2 (Sumpftemperatur 170°C) in zwei Schritten über Kopf und TDI jeweils über Sumpf abgezogen. In der Kolonne K3 (Sumpftemperatur 150°C) wurde TDI über Kopf genommen, die Schwerprodukte konzentriert über Sumpf abgezogen und in einem Schaufeltrockner (List Reaktor) eingeengt. Die Ausbeute des Verfahrens betrug 95 w/w % TDI. Die mittlere Temperaturbelastung in den drei Kolonnensümpfen K1, K2 und K3 betrug 160°C. bei einer summierten Verweilzeit von 4 Stunden. In den Kolonnensümpfen gingen 3,5 w/w % Ausbeute durch die Temperaturbelastung und den damit verbundenen Ausbeuteverlust durch Isocyanuratbildung verloren.
Die Viskosität des Sumpfes von K3 betrug bei 130°C ca. 800 mPas (25w% TDI)

### Beispiel 2 (erfindungsgemäß):

TDI wurde wie in Beispiel 1 aus TDA und Phosgen synthetisiert. Der Reaktionsaustrag enthielt 77% TDI, 20% Chlorbenzol und 3% nicht verdampfbaren Rückstand. In einem Wendelrohrverdampfer W1 wurden TDI und Lösungsmittel vor der K1 über Kopf gezogen. Die Eindampfrate betrug 96,5%. Der Druck im Abscheider betrug 5 mbar. Die Ausbeute des Verfahrens betrug jetzt 96, 2%. Die Viskosität des auf 80°C gekühlten Sumpfproduktes betrug nur 400 mPas (20w% TDI) und war damit besser handhabbar.

Das Lösungsmittel wurde in den Kolonnen K1 und K2, die unter den Bedingungen wie in Beispiel 1 beschrieben arbeiteten, wieder in zwei Schritten über Kopf und TDI über Sumpf abgezogen. In der Kolonne K3, die ebenfalls unter den Bedingungen wie in Beispiel 1 arbeitete, wurde TDI über Kopf genommen, und eine jetzt sehr geringe Menge aus TDI und Schwerprodukt wurden konzentriert über Sumpf der K3 abgezogen und zusammen mit dem Rückstand des Wendelrohrverdampfers in einem Schaufeltrockner (List Reaktor) eingeengt. In den Kolonnensümpfen der K1, K2 und K3 war die Konzentration an Nebenkomponenten so gering, dass keine nennenswerte ausbeutemindemde Schwerproduktbildung mehr auftrat.

Der Sumpf des Wendelrohrverdampfers wurde einem Knetreaktor (List Reaktor) bei 40 mbar und 240°C zugeführt. Die Verweilzeit betrug ca. 2-3 Stunden. Im Zulauf befanden sich 20% TDI und 80% Destillationsrückstand. Am Feststoffaustrag des Knetreaktors erhielt man lediglich einen Rückstand, der 40% der eingesetzten Menge enthielt. Der Brüden enthielt mehr als 99 w/w% TDI.

### Beispiel 3 (erfindungsgemäß):

TDI wurde aus TDA und Phosgen wie in Beispiel 1 synthetisiert. Der Reaktionsaustrag enthielt 77% TDI, 20% Chlorbenzol und 3% nicht verdampfbaren Rückstand. Im Wendelrohrverdampfer W1 wurden TDI und Lösungsmittel über Kopf gezogen. Die Eindampfrate betrug 96,5%. Der Druck im Abscheider betrug 5 mbar. Die Ausbeute des Verfahrens betrug jetzt 96,2%. Die Viskosität des auf 80°C gekühlten Sumpfproduktes betrug nur 400 mPas (20w% TDI) und war damit besser handhabbar.

Das Lösungsmittel wurde in den Kolonnen K1 und K2, die unter den Bedingungen wie in Beispiel 1 beschrieben arbeiteten, wieder in zwei Schritten über Kopf und TDI über Sumpf abgezogen. In der Kolonne K3, die unter den Bedingungen wie in Beispiel 1 beschrieben arbeitete, wurde TDI über Kopf genommen, eine jetzt sehr geringe Menge aus TDI und Schwerprodukt wurden konzentriert über den Sumpf der K3 abgezogen und zusammen mit dem Rückstand des Wendelrohrverdampfers in einem Umlaufverdampfer mit Brüdenabscheider eingeengt. In den Kolonnensümpfen der K1, K2 und K3 war die Konzentration an Nebenkomponenten jetzt so gering, dass keine nennenswerte Schwerproduktbildung mehr auftrat.

Die Verweilzeit im Umlaufverdampfer betrug ca. 1,5 Stunden. Die Sumpftemperatur betrug 150°C. Im Zulauf befanden sich 20% TDI und 80% Destillationsrückstand. Der Rückstand wurde am Eingang des Verdampfers 1:1 w/w mit Dichlorbenzol verdünnt.

Am Kopf des Umlaufverdampfers wurde eine Mischung aus Dichlorbenzol und TDI abgezogen. Am Sumpf des Umlaufverdampfers wurde eine Mischung aus TDI und Rückstand abgezogen.
Durch die thermische Behandlung wurden 20-25 w/w % des Rückstands in TDI umgewandelt.

### Beispiel 4 (erfindungsgemäß):

TDI wurde wie in Beispiel 1 aus TDA und Phosgen synthetisiert. Der Reaktionsaustrag enthielt 77% TDI, 20% Chlorbenzol und 3% nicht verdampfbaren Rückstand. Im Wendelrohrverdampfer W1 wurden TDI und Lösungsmittel über Kopf gezogen. Die Eindampfrate betrug 96,5%. Der Druck im Abscheider betrug 5 mbar. Die Ausbeute des Verfahrens betrug jetzt 96,2%. Die Viskosität des auf 80°C gekühlten Sumpfproduktes betrug nur 400 mPas (20w% TDI) und war damit besser handhabbar.

Das Lösungsmittel wurde in den Kolonnen K1 und K2, die unter den Bedingungen wie in Beispiel 1 beschrieben arbeiteten, wieder in zwei Schritten über Kopf und TDI über Sumpf abgezogen. In der Kolonne K3, die unter den Bedingungen wie in Beispiel 1 beschrieben arbeitete, wurde TDI über Kopf genommen und eine jetzt sehr geringe Menge aus TDI und Schwerprodukte wurde konzentriert über Sumpf abgezogen und zusammen mit dem Rückstand des Wendelrohrverdampfers in einem Umlaufverdampfer mit Brüdenabscheider eingeengt. In den Kolonnensümpfen der Kolonnen K1, K2 und K3, war die Konzentration an Nebenkomponenten so gering, dass keine nennenswerte Schwerproduktbildung mehr auftrat.

Die Verweilzeit im Umlaufverdampfer betrug ca. 1,5 Stunden. Die Sumpftemperatur betrug 150°C. Im Zulauf befanden sich 20% TDI und 80% Destillationsrückstand. Der Rückstand wurde am Eingang des V. 1:1 w/w mit Phtalsäuredibutylester verdünnt.

Am Kopf des Umlaufverdampfers wird TDI abgezogen. Am Sumpf des Umlaufverdampfers wurde eine Mischung aus TDI und Phtalsäuedibutylester abgezogen. Durch die thermische Behandlung wurden 20-25 w/w % des Rückstands in TDI umgewandelt.

## Patentansprüche

1. Verfahren zur Herstellung von Polyisocyanaten, umfassend die Schritte
a) Umsetzung von Aminen mit Phosgen,
b) Abtrennung von Chlorwasserstoff, überschüssigem Phosgen und gegebenenfalls des Lösungsmittels aus dem Reaktionsgemisch,
c) Trennung des flüssigen Gemisches aus Schritt b) in eine flüssige und eine gasförmige Phase in einem Wendelrohrverdampfer.
d) Aufarbeitung der gasförmigen Phase aus Schritt c) zum Polyisocyanat.

2. Verfahren nach Anspruch 1, **dadurch gekennzeichnet, dass** das Gemisch aus Schritt b) vor dem Schritt c) unter Druck vorgewärmt und bei Eintritt in den Apparat für Schritt c) entspannt wird.

3. Verfahren nach Anspruch 1, **dadurch gekennzeichnet, dass** der Druck nach dem Apparat für Schritt c) im Abscheider 5 bis 200 mbar beträgt.

4. Verfahren nach Anspruch 1, **dadurch gekennzeichnet, dass** der Druck nach dem Apparat für Schritt c) im Abscheider 5 bis 30 mbar beträgt.

5. Verfahren nach Anspruch 1, **dadurch gekennzeichnet, dass** die Temperatur am Austritt des Apparats für Schritt c) 100 bis 300 °C beträgt.

6. Verfahren nach Anspruch 1, **dadurch gekennzeichnet, dass** die Temperatur am Austritt des Apparats für Schritt c) 130 bis 250 °C beträgt.

7. Verfahren nach Anspruch 1, **dadurch gekennzeichnet, dass** die gasförmige Phase aus Schritt c) kondensiert und in Schritt d) einer Destillation unterzogen wird.

8. Verfahren nach Anspruch 1, **dadurch gekennzeichnet, dass** die flüssige Phase aus Schritt c) thermisch behandelt wird.

9. Verfahren nach Anspruch 1, **dadurch gekennzeichnet, dass** die thermische Behandlung in einem Verweilzeitraum durchgeführt wird.

10. Verfahren nach Anspruch 1 **dadurch gekennzeichnet, dass** der Verweilzeitraum ein Behälter, Schaufeltrockner, Rührkessel, Rohrreaktor, Wendelrohrverdampfer, Wärmetauscher, Kolonnensumpf, Extruder oder Kneter ist

11. Verfahren nach Anspruch 1, **dadurch gekennzeichnet, dass** die thermische Behandlung in einem Wendelrohrverdampfer durchgeführt wird.

12. Verfahren nach Anspruch 1, **dadurch gekennzeichnet, dass** die thermische Behandlung bei einer Temperatur von 120-250°C durchgeführt wird.

13. Verfahren nach Anspruch 1, **dadurch gekennzeichnet, dass** die thermische Behandlung bei einer Temperatur von 150-240°C durchgeführt wird.

14. Verfahren nach Anspruch 1, **dadurch gekennzeichnet, dass** die flüssige Phase aus Schritt c) einem Dünnschichtverdampfer oder Kurzwegverdampfer zugeführt wird.

15. Verfahren nach Anspruch 1, **dadurch gekennzeichnet, dass** die flüssige Phase aus Schritt c) einer Hydrolyse zugeführt und das dort gewonnene Amin in Schritt a) zurückgeführt wird.

16. Verfahren nach Ansprüchen 1-12, **dadurch gekennzeichnet, dass** das Polyisocyanat ausgewählt ist aus der Gruppe, enthaltend Toluylendiisocyanat, Diphenylmethandiisocyanat, Isophorondiisocyanat und Hexamethylendiisocyanat.

17. Verfahren nach Ansprüchen 1-12, **dadurch gekennzeichnet, dass** das Polyisocyanat Toluylendiisocyanat ist.

## Claims

1. A process for preparing polyisocyanates, comprising the steps of
a) reacting amines with phosgene,
b) removing hydrogen chloride, excess phosgene and, if appropriate, the solvent from the reaction mixture,
c) separating the liquid mixture from step b) into a liquid phase and a gaseous phase in a helical tube evaporator,
d) working up the gaseous phase from step c) to give the polyisocyanate.

2. The process according to claim 1, wherein the mixture from step b), before step c), is preheated under pressure and decompressed on entry into the apparatus for step c).

3. The process according to claim 1, wherein the pressure downstream of the apparatus for step c) in the separator is from 5 to 200 mbar.

4. The process according to claim 1, wherein the pressure downstream of the apparatus for step c) in the separator is from 5 to 30 mbar.

5. The process according to claim 1, wherein the temperature at the exit of the apparatus for step c) is from 100 to 300°C.

6. The process according to claim 1, wherein the temperature at the exit of the apparatus for step c) is from 130 to 250°C.

7. The process according to claim 1, wherein the gaseous phase from step c) is condensed and subjected to a distillation in step d).

8. The process according to claim 1, wherein the liquid phase from step c) is treated thermally.

9. The process according to claim 1, wherein the thermal treatment is performed in a delay zone.

10. The process according to claim 1, wherein the delay zone is a vessel, paddle drier, stirred tank, tubular reactor, helical tube evaporator, heat exchanger, column bottom, extruder or kneader.

11. The process according to claim 1, wherein the thermal treatment is performed in a helical tube evaporator.

12. The process according to claim 1, wherein the thermal treatment is performed at a temperature of 120-250°C.

13. The process according to claim 1, wherein the thermal treatment is performed at a temperature of 150-240°C.

14. The process according to claim 1, wherein the liquid phase from step c) is sent to a thin-film evaporator or short-path evaporator.

15. The process according to claim 1, wherein the liquid phase from step c) is sent to a hydrolysis and the amine recovered there is recycled into step a).

16. The process according to claims 1-12, wherein the polyisocyanate is selected from the group comprising tolylene diisocyanate, diphenylmethane diisocyanate, isophorone diisocyanate and hexamethylene diisocyanate.

17. The process according to claims 1-12, wherein the polyisocyanate is tolylene diisocyanate.

## Revendications

1. Procédé pour la production de polyisocyanates, comprenant les étapes
a) mise en réaction d'amines avec du phosgène,
b) séparation de chlorure d'hydrogène, phosgène en excès et éventuellement du solvant d'avec le mélange réactionnel,
c) fractionnement du mélange liquide provenant de l'étape b) en une phase liquide et une phase gazeuse dans un évaporateur à tubes hélicoïdaux,
d) traitement final de la phase gazeuse provenant de l'étape c), pour l'obtention de polyisocyanate.

2. Procédé selon la revendication 1, **caractérisé en ce que** le mélange provenant de l'étape b) est préchauffé sous pression avant l'étape c) et détendu lors de l'entrée dans l'appareil pour l'étape c).

3. Procédé selon la revendication 1, **caractérisé en ce que** la pression après l'appareil pour l'étape c) vaut de 5 à 200 mbars dans le séparateur.

4. Procédé selon la revendication 1, **caractérisé en ce que** la pression après l'appareil pour l'étape c) vaut de 5 à 30 mbars dans le séparateur.

5. Procédé selon la revendication 1, **caractérisé en ce que** la température à la sortie de l'appareil pour l'étape c) vaut de 100 à 300 °C.

6. Procédé selon la revendication 1, **caractérisé en ce que** la température à la sortie de l'appareil pour l'étape c) vaut de 130 à 250 °C.

7. Procédé selon la revendication 1, **caractérisé en ce que** la phase gazeuse provenant de l'étape c) est condensée et soumise à une distillation dans l'étape d).

8. Procédé selon la revendication 1, **caractérisé en ce que** la phase liquide provenant de l'étape c) est traitée thermiquement.

9. Procédé selon la revendication 1, **caractérisé en ce que** le traitement thermique est effectué dans une chambre à temps de séjour.

10. Procédé selon la revendication 1, **caractérisé en ce que** la chambre à temps de séjour est un récipient, un sécheur à ailettes, une cuve à agitation, un réacteur tubulaire, un évaporateur à tubes hélicoïdaux, un échangeur thermique, le fond d'une colonne, une extrudeuse ou un malaxeur.

11. Procédé selon la revendication 1, **caractérisé en ce que** le traitement thermique est effectué dans un évaporateur à tubes hélicoïdaux.

12. Procédé selon la revendication 1, **caractérisé en ce que** le traitement thermique est effectué à une température de 120-250 °C.

13. Procédé selon la revendication 1, **caractérisé en ce que** le traitement thermique est effectué à une température de 150-240 °C.

14. Procédé selon la revendication 1, **caractérisé en ce que** la phase liquide provenant de l'étape c) est envoyée à un évaporateur à couche mince ou un évaporateur à trajet court.

15. Procédé selon la revendication 1, **caractérisé en ce que** la phase liquide provenant de l'étape c) est envoyée à une hydrolyse et l'amine qui y est recueillie est recyclée dans l'étape a).

16. Procédé selon les revendications 1-12, **caractérisé en ce qu'**on choisit le polyisocyanate dans le groupe contenant le toluylène-diisocyanate, le diphénylméthane-diisocyanate, l'isophorone-diisocyanate et l'hexaméthylène-diisocyanate.

17. Procédé selon les revendications 1-12, **caractérisé en ce que** le polyisocyanate est le toluylène-diisocyanate.
